# EUROPÄISCHE PATENTSCHRIFT

(11) **EP 2 750 617 B1**
(45) Veröffentlichungstag und Bekanntmachung des Hinweises auf die Patenterteilung: **21.11.2018**
(21) Anmeldenummer: 12768702.8
(22) Anmeldetag: 24.08.2012
(51) Int. Cl.: A61B 17/80, A61B 17/56, A61F 2/42

(54) **ANATOMISCH ANGEPASSTE, PLANTARE KNOCHENPLATTE SOWIE KNOCHENPLATTENSYSTEM**
ANATOMICALLY CUSTOMIZED PLANTAR BONE PLATE AND BONE PLATE SYSTEM
PLAQUE OSSEUSE PLANTAIRE, ANATOMIQUEMENT ADAPTÉE, ET SYSTÈME DE PLAQUE OSSEUSE

(30) Priorität: 31.08.2011 DE 202011051165 U
(43) Veröffentlichungstag der Anmeldung: 09.07.2014
(73) Patentinhaber: Aristotech Industries GmbH, 14943 Luckenwalde (DE)
(72) Erfinder: GAUDIN, Bernd, P., 14942 Luckenwalde (DE)
(74) Vertreter: Bittner, Thomas L.
(86) Internationale Anmeldenummer: PCT/DE2012/100248
(87) Internationale Veröffentlichungsnummer: WO 2013/029600

(56) Entgegenhaltungen:
- EP-A2- 1 468 655
- US-A1- 2006 173 458
- US-A1- 2008 300 637

## Beschreibung

Die Erfindung betrifft eine anatomisch angepasste, plantare Knochenplatte zur winkelstabilen Fixation für die Arthrodese des Tarsometatarsal-I-Gelenks sowie ein Knochenplattensystem.

### Hintergrund der Erfindung

Derartige Knochenplatten dienen zur Fixation des Tarsometatarsal-I-Gelenks im Rahmen einer Gelenkversteifung. Angestrebt ist eine mechanische Stabilisierung der Knochenteile zueinander, die es einem Patienten zum Beispiel nach einer Operation ermöglicht, recht schnell den Fuß wieder zu belasten. Bekannte Knochenplatten für die Arthrodese des Tarsometatarsal-I-Gelenks verfügen über mehrere Bohrungen, in die jeweils eine Knochenschraube winkelstabil eingeschraubt werden kann.

In dem Dokument WO 2010/059497 A1 ist eine dorsale Knochenplatte zur winkelstabilen Fixation für die Arthrodese des Tarsometatarsal-I-Gelenks offenbart. In einem proximalen Plattenabschnitt der Knochenplatte ist eine Gruppe von drei Bohrungen gebildet, die jeweils der winkelstabilen Aufnahme einer Knochenschraube dienen. Auch der distale Plattenabschnitt der Knochenplatte ist mit drei Bohrungen zur winkelstabilen Aufnahme von Knochenschrauben versehen. Darüber hinaus ist im distalen Abschnitt ein Längsloch zur winkelflexiblen Aufnahme einer weiteren Knochenschraube vorgesehen. In einer Ausgestaltung kann im Bereich zwischen dem proximalen und dem distalen Plattenabschnitt eine Biegung vorgesehen sein. Die dorsale Positionierung der Knochenplatte hat den Nachteil, dass hierdurch nur eine geringe Weichteildeckung am Fuß gegeben ist. Auch tritt hierbei eine hohe Biegebelastung für die Knochenplatte auf. Bei Fußbelastung kommt es zum Klaffen des Tarsometatarsal-I-Gelenkspaltes.

Aus dem Dokument DE 10 2009 020 285 A1 ist eine Knochenplatte zum Fixieren von Knochenteilen bei operativen Korrekturen der Hallux Valgus Deformität bekannt, die im Wesentlichen L-förmig oder T-förmig ausgebildet ist. Die L- oder T-Schenkel weisen Durchgangslöcher zur Aufnahme der Köpfe von Knochenschrauben auf.

Im Dokument EP 1 897 509 B1 ist eine Knochenplatte mit mehreren Durchbrüchen zur Aufnahme einer oder mehrerer Knochschrauben offenbart.

Im Dokument US 2006/0015102 A1 ist eine Knochenplatte zur Osteosynthese von Knochfragmenten offenbart. Die Knochplatte weist ein Langloch und mehrere Bohrungen auf.

Auch im Dokument US 2010/0256687 A1 ist eine Knochenplatte beschrieben, bei der sich endseitig jeweils ein Endabschnitt schräg zur Längsachse der Knochplatte erstreckt. Sowohl im mittleren Bereich als auch in den abgewinkelten Endabschnitten sind Durchbrüche für Knochenschrauben hergestellt.

Das Dokument WO 2010/059497 A1 offenbart eine Knochplatte mit einem Langloch und mehreren weiteren Durchbrüchen zur Aufnahme von Schrauben, insbesondere Knochenschrauben.

Das Dokument US 2008/0300637 A1 offenbart verschiedene Knochenplatten, die an unterschiedlichen Knochen angeordnet sein können.

Das Dokument US 2006/0173458 A1 offenbart ein Haltesystem für Knochen bei einer Fraktur.

### Zusammenfassung der Erfindung

Aufgabe der Erfindung ist es, verbesserte Technologien in Verbindung mit einer Knochenplatte zur winkelstabilen Fixation für die Arthrodese des Tarsometatarsal-I-Gelenks anzugeben, mit denen eine optimierte mechanische Stabilisierung der Gelenkversteifung ermöglich ist. Darüber hinaus soll die Implantierbarkeit der Knochenplatte erleichtert werden.

Diese Aufgabe wird erfindungsgemäß durch eine anatomisch angepasste, plantare Knochenplatte zur winkelstabilen Fixation für die Arthrodese des Tarsometatarsal-I-Gelenks nach dem unabhängigen Anspruch 1 gelöst. Weiterhin ist ein Knochenplattensystem für die Arthrodese des Tarsometatarsal-I-Gelenks nach dem unabhängigen Anspruch 7 geschaffen. Vorteilhafte Ausgestaltungen der Erfindung sind Gegenstand von abhängigen Unteransprüchen.

Die Erfindung umfasst den Gedanken einer anatomisch angepassten, plantaren Knochenplatte zur winkelstabilen Fixation für die Arthrodese des Tarsometatarsal-I-Gelenks mit einem distalen Plattenabschnitt, der sich in Längsrichtung erstreckt und in welchem eine distale Bohrung zum winkelstabilen Aufnehmen einer Knochenschraube gebildet ist, einem proximalen Plattenabschnitt, der sich quer zur Längsrichtung erstreckt, und einem mittleren Plattenabschnitt, welcher sich zwischen dem distalen und dem proximalen Plattenabschnitt erstreckt und zur anatomischen Anpassung mit einem gebogenen Plattenabschnitt gebildet ist, wobei im proximalen Plattenabschnitt quer zur Längsrichtung nebeneinander eine plantare Bohrung zum winkelstabilen Aufnehmen einer plantar zu fixierenden Knochenschraube und eine nach medial abgewinkelte Bohrung zum winkelstabilen Aufnehmen einer nach medial abgewinkelt zu fixierenden Knochenschraube angeordnet sind. Gemäß der Erfindung ist auch ein Knochenplattensystem für die Arthrodese des Tarsometatarsal-I-Gelenks geschaffen mit einer anatomisch angepassten, plantaren Knochenplatte, bei der eine distaler Plattenabschnitt, der sich in Längsrichtung erstreckt und in welchem eine distale Bohrung zum winkelstabilen Aufnehmen einer Knochenschraube gebildet ist, eine proximaler Plattenabschnitt, der sich quer zur Längsrichtung erstreckt, und eine mittlerer Plattenabschnitt gebildet sind, welcher sich zwischen dem distalen und dem proximalen Plattenabschnitt erstreckt und zur anatomischen Anpassung mit einem gebogenen Plattenabschnitt ausgeführt ist, wobei im proximalen Plattenabschnitt quer zur Längsrichtung nebeneinander eine plantare Bohrung zum winkelstabilen Aufnehmen einer plantar zu fixierenden Knochenschraube und eine mediale Bohrung zum winkelstabilen Aufnehmen einer nach medial abgewinkelt zu fixierenden Knochenschraube angeordnet sind, und mehreren Knochenschrauben zum jeweils winkelstabilen Einschrauben in die distale Bohrung, die plantare Bohrung sowie die nach medial abgewinkelte Bohrung.
Die nach medial abgewinkelte Bohrung ist an einem seitlich abstehenden Abschnitt des proximalen Plattenabschnittes gebildet. Der seitlich abstehende Abschnitt der Knochenplatte kann zum Beispiel mit einer seitlichen Lasche gebildet sein. Die Ausgestaltung, bei der der seitlich abstehende Abschnitt am proximalen Plattenabschnitt gebildet ist, weist die Knochenplatte bevorzugt eine L-Form auf.
Der seitlich abstehende Abschnitt ist gegenüber einem mittleren Abschnitt des proximalen Plattenabschnittes zur Plattenunterseite hin geneigt. Der seitliche Abschnitt ist in Blickrichtung in Längsrichtung gegenüber dem mittleren Abschnitt im Bereich des proximalen Plattenabschnittes hierbei zur Unterseite hin umgebogen oder abgewinkelt.

Der seitlich abstehende Abschnitt ist mit einer Schränkung versehen. Eine solche Schränkung ist vergleichbar dem Schränken von Sägezähnen ausgeführt. Die Schränkung ist bevorzugt mit einem Schränkungswinkel von etwa 3 bis etwa 10 Grad gebildet. Bei einer bevorzugten Ausgestaltung ist ein Eckbereich des seitlich abstehenden Abschnittes geschränkt, welcher auf der dem distalen Plattenabschnitt zugewandten Seite des seitlich abstehenden Abschnittes angeordnet ist.

Der mittlere Plattenabschnitt ist frei von Schraubenbohrungen. Hierdurch wird die mechanische Stabilität der Knochenplatte weiter unterstützt.

Der mittlere Plattenabschnitt weist eine proximale Biegezone auf, die dem Gelenkspalt des zu fixierenden Gelenks zugeordnet ist. Die proximale Biegezone oder Biegung ist derart ausgestaltet, dass sich an einen im Wesentlichen ebenen Plattenabschnitt, der im Bereich des proximalen Plattenabschnitts beginnt und sich zu dem mittleren Plattenabschnitt hin fortsetzt, die proximale Biegezone anschließt. Die proximale Biegung führt dann in den mittleren Plattenabschnitt über, der eben oder in Blickrichtung von der Seite auf die Knochenplatte durchgebogen ausgeführt sein kann. Der mittlere Plattenabschnitt koppelt danach über eine weitere Biegezone oder Biegung an den distalen Plattenabschnitt, der in dieser oder anderen Ausgestaltungen als ebener Plattenabschnitt oder in Blickrichtung von der Seite auf die Knochenplatte durchgebogener Plattenabschnitt ausgeführt sein kann. Die eine oder die mehreren Biegezonen oder Biegungen ermöglichen eine optimierte Anpassung der Knochenplattenform an die anatomischen Verhältnisse.

Die Biegezonen im mittleren Plattenabschnitt sind jeweils mit einer Materialverdünnung gebildet sind. Die Materialverdünnungen bilden Taillierungen, die vor der tatsächlichen Implantation wahlweise zur ergänzenden Formanpassung der Knochenplatte an anatomische Verhältnisse nutzbar sind, indem bei der Herstellung vorgegebene Biegungen verändert werden, sei es von Hand oder unter Nutzung eines Werkzeuges.

Die vorgeschlagene plantare Knochenplatte unterstützt die Stabilität der operativ mittels der Knochenplatte bewirkten Gelenkversteifung, indem sie im proximalen Plattenbereich mittels zweier nebeneinander angeordneter Knochenschrauben fixierbar ist, von denen eine Knochenschraube plantar und eine andere Knochenschraube medial am Knochen zu fixieren sind. Es werden auf diese Weise nicht nur die plantar günstigeren Kraftverhältnisse am Tarsometatarsal-I-Gelenk genutzt, sondern zusätzlich eine mediale Fixationskomponente der Knochenplatte ermöglicht. Hierdurch ist die mechanische Stabilität der winkelstabilen Fixation am Knochen verbessert.

Darüber hinaus besteht der Vorteil, dass beim Implantieren der Knochenplatte diese zunächst von medial aus fixiert werden kann, um anschließend die weiteren Knochenschrauben im schwerer zugänglichen plantaren Bereich einzuschrauben. Hierdurch ist für den Operateur eine positionsgenaue Fixierung der Knochenplatte relativ zum versteifenden Gelenk erleichtert. Es ist die Möglichkeit geschaffen, die plantar vorteilhafteren Verhältnisse der Kraftwirkung auf die Knochenplatte am zu versteifenden Gelenk trotz der hier unter Umständen schwierigeren Implantation zu nutzen, indem mit Hilfe der vorgeschlagenen anatomisch angepassten, plantaren Knochenplatte eine ortsgenaue Fixation mit ausreichender mechanischer Stabilität ermöglicht ist.

Das winkelstabile Fixieren der Knochenschrauben in der jeweils zugeordneten Bohrung kann auf verschiedene Art und Weise erfolgen. Hier sind unterschiedliche Ausgestaltungen als solche bekannt. Beispielsweise ist die Bohrung mit einem Gewindeabschnitt versehen, in welchen sich beim Einschrauben der Knochenschraube ein zugeordnetes Gewinde am Schraubenkopf eindreht, wodurch im eingedrehten Zustand der Knochenschraube eine Winkelstabilität gesichert ist.

In einer Ausgestaltung kann vorgesehen sein, dass die Knochenplatte umlaufend mit einer Phase oder einer Verrundung versehen ist, um nachteilige Wirkungen auf das umgebende Gewebe im Körper zu vermeiden.

Die anatomisch angepasste Ausführung der plantaren Knochenplatte bedeutet, dass die Knochenplatte bei der Herstellung vorgeformt wird, so dass die Knochenplatte nach der Herstellung eine an die anatomischen Verhältnisse im Bereich des Tarsometatarsal-I-Gelenks angepasste Form aufweist.

Bei einer vorteilhaften Ausgestaltung der Erfindung kann vorgesehen sein, dass mittels des distalen und des proximalen Plattenabschnitts und des sich hierzwischen erstreckenden mittleren Plattenabschnitts in Blickrichtung von der Seite eine S-Plattenkontur gebildet ist.

Eine bevorzugte Weiterbildung der Erfindung sieht vor, dass die plantare Bohrung und die nach medial abgewinkelte Bohrung mit sich auf der Plattenunterseite kreuzenden Einschraubachsen für die plantar und die nach medial abgewickelt zu fixierende Knochenschraube gebildet sind. Die Kreuzung der Einschraubachsen, die zum Beispiel mittels eines Gewindeabschnitts in der Bohrung vorgegeben sind, ist insbesondere in Blickrichtung quer zur Längsrichtung einsehbar. Mittels Überkreuzen der Einschraubachsen wird der Widerstand gegen ein Abziehen der Platte deutlich vergrößert. Ausreißkräfte steigen, da nicht nur die Gewinde eine Verankerung darstellen, sondern auch die kreuzenden Schrauben als Bolzen dienen.

Bei einer zweckmäßigen Ausgestaltung der Erfindung kann vorgesehen sein, dass im distalen Plattenabschnitt in Längsrichtung benachbart zu der distalen Bohrung eine weitere distale Bohrung zum winkelstabilen Aufnehmen einer weiteren Knochenschraube gebildet ist. Wie die Knochenschraube für die distale Bohrung ist auch die weitere Knochenschraube für die weitere distale Bohrung beim winkelstabilen Fixieren der Knochenplatte plantar einzuschrauben.

Eine vorteilhafte Ausführungsform der Erfindung sieht ein oder mehrere Durchbrüche zum winkelstabilen Aufnehmen eines Kirschner-Drahtes vor. Derartige Drähte werden auch als K-Drähte bezeichnet. Sie dienen in der Chirurgie als Hilfsmittel, beispielsweise zur provisorischen Lagefixierung.

Bevorzugt sieht eine Fortbildung der Erfindung vor, dass eine Einsteckachse eines Durchbruchs, der im proximalen Plattenabschnitt oder benachbart hierzu gebildet ist, im Wesentlichen achsenparallel zur Einschraubachse für die plantare Knochenschraube in der plantaren Bohrung verläuft. Die Einsteckachse entspricht der Achse der Bohrung, mit der der Durchbruch ausgebildet wurde. Entlang dieser Achse wird der Kirschner-Draht im Durchbruch beim Einstecken oder Einführen in den Durchbruch geführt. Es kann vorgesehen sein, dass ergänzend oder alternativ eine Einsteckachse eines weiteren Durchbruchs, der im distalen Plattenabschnitt oder benachbart hierzu gebildet ist, im Wesentlichen achsenparallel zur Einschraubachse der in die proximale Bohrung plantar einzuschraubende Knochenschraube gebildet ist. Mit Hilfe der Achsenparallelität ist es dem Operateur jeweils ermöglicht, zunächst einen K-Draht in die entsprechende Bohrung einzuführen und so eine Orientierung für die Einschraubrichtung der zugeordneten Knochenschraube zu haben. Auch kann später auf Röntgenbildern die angestrebte Parallelität zwischen provisorisch eingeführtem K-Draht und eingeschraubter Knochenschraube nachgeprüft werden.
Mit Hilfe der vorgeschlagenen Knochenplatte sowie des geschaffenen Knochenplattensystems für die Arthrodese des Tarsometatarsal-I-Gelenks ist ein Verfahren zum Implantieren der Knochenplatte ermöglicht, bei dem in eine zugeordnete Körperöffhung die anatomisch angepasste, plantare Knochenplatte eingeführt und im Bereich des Tarsometatarsal-I-Gelenks angeordnet wird, die Knochenplatte (provisorisch) fixiert wird, indem in die nach medial abgewinkelte Bohrung eine zugeordnete Knochenschraube eingeschraubt wird und anschließend weitere Knochenschrauben in die proximale Bohrung sowie die plantare Bohrung eingeschraubt werden, wodurch sämtliche Knochenschrauben winkelstabil in der jeweils zugeordneten Bohrung aufgenommen sind. Es kann vorgesehen sein, ergänzend dorsal eine Zugschraube in die mittels der Knochenplatte fixierten Knochenteile des Gelenks einzuschrauben. Mit Hilfe dieses Verfahrens ist es dem Operateur ermöglicht, die Knochenplatte zunächst mittels der nach medial abgewinkelt zu fixierenden Knochenschraube zu positionieren und zu befestigen, also mittels Einbringen einer Knochenschraube in einem operativ leichter zugänglichen Bereich, nämlich dem medialen Mittelfußbereich. Anschließend können dann die weiteren Knochenschrauben plantar eingeschraubt werden. Das Verfahren sieht vor, eine proximale Biegung des zur anatomischen Anpassung gebogenen Plattenabschnitts benachbart zum (ehemaligen) Gelenkspalt anzuordnen.

### Beschreibung von bevorzugten Ausführungsbeispielen der Erfindung

Die Erfindung wird im Folgenden anhand von bevorzugten Ausführungsbeispielen unter Bezugnahme auf Figuren einer Zeichnung näher erläutert. Hierbei zeigen:
- Fig. 1: eine anatomisch angepasste, plantare Knochenplatte zur winkelstabilen Fixation für die Arthrodese des Tarsometatarsal-I-Gelenks in Draufsicht,
- Fig. 2: die Knochenplatte aus Fig. 1 in Seitenansicht,
- Fig. 3: eine schematische Darstellung eines Abschnitts eines Fußskeletts, bei dem am Tarsometatarsal-I-Gelenk die Knochenplatte nach Fig. 1 und Fig. 2 winkelstabil fixiert ist,
- Fig. 4: eine schematische Darstellung des Fußskelettabschnitts aus Fig. 3 aus einer weiteren Perspektive und
- Fig. 5: eine schematische Darstellung des Fußskelettabschnitts aus Fig. 3 aus einer anderen Perspektive.

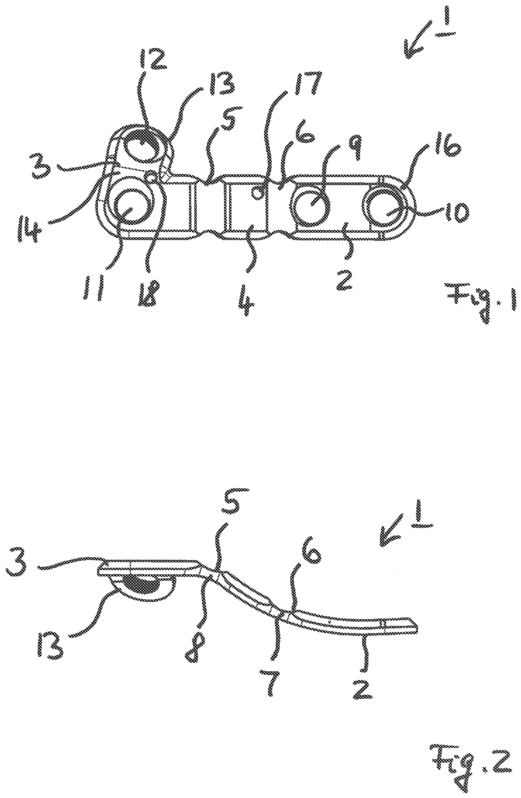

Fig. 1 zeigt eine anatomisch angepasste, plantare Knochenplatte 1, die mit einem distalen Plattenabschnitt 2 und einem proximalen Plattenabschnitt 3 gebildet ist. Zwischen dem distalen Plattenabschnitt 2 und dem proximalen Plattenabschnitt 3 ist ein mittlerer Plattenabschnitt 4 angeordnet, der gemäß Fig. 2 im Bereich von Materialtaillierungen 5, 6 mit einer distalen Biegezone 7 sowie einer proximalen Biegezone 8 versehen ist, so dass die Knochenplatte 1 in Blickrichtung von der Seite mit einer S-Form oder S-Kontur gebildet ist. Die S-Kontur ist Teil der anatomischen Anpassung der Knochenplatte 1.

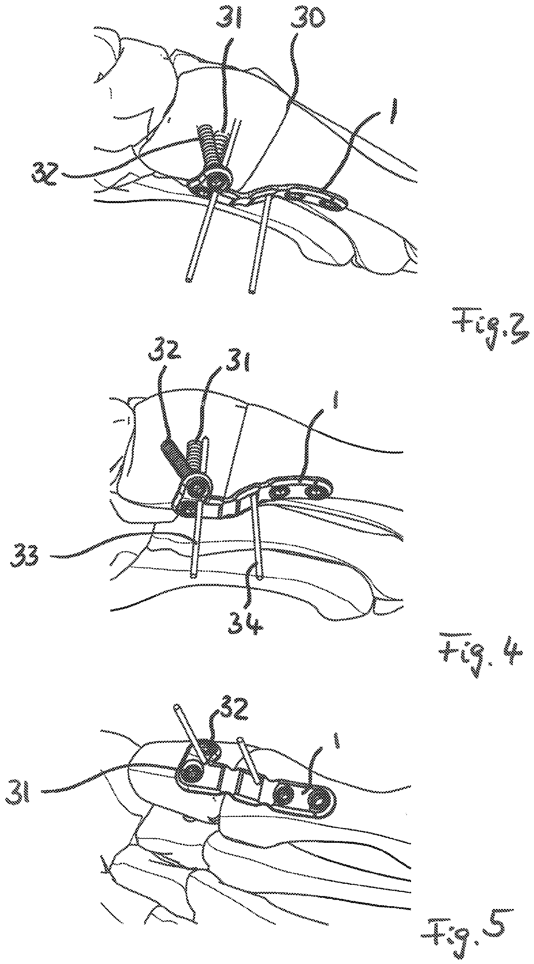

Gemäß Fig. 1 sind im distalen Plattenabschnitt 2 eine distale Bohrung 9 sowie eine weitere distale Bohrung 10 in Längsrichtung des distalen Plattenabschnitts 2 nebeneinander angeordnet. Die distale Bohrung 9 und die weitere distale Bohrung 10 dienen beide der winkelstabilen Aufnahme einer zugehörigen Knochenschraube, was sich weitergehend aus den Fig. 3 bis 5 unten ergibt.

In vergleichbarer Weise nehmen im proximalen Plattenabschnitt 3 eine plantare Bohrung 11 sowie eine nach medial abgewinkelte Bohrung 12 zugeordnete Knochenschrauben winkelstabil auf, was ebenfalls unten in den Fig. 3 bis 5 gezeigt ist. Wie sich am besten aus Fig. 2 ergibt, ist eine Lasche 13 im Bereich des proximalen Plattenabschnitts 3 mit der nach medial abgewinkelten Bohrung 12 versehen und im Vergleich zu einem benachbarten Abschnitt 14 im proximalen Plattenabschnitt 3 abgewinkelt zur Unterseite der Knochenplatte 1. Darüber hinaus ist die Lasche 13 geschränkt, nämlich mit einem Winkel von etwa 5 Grad. Die dreidimensionale Ausgestaltung des proximalen Plattenabschnitts 3 bildet eine weitere Maßnahme zur anatomischen Anpassung der Knochenplatte 1 an die anatomischen Verhältnisse im Bereich des Tarsometatarsal-I-Gelenks. Die beschriebenen Merkmale der anatomischen Anpassung der Knochenplatte 1 werden bereits bei der Herstellung der Knochenplatte 1 ausgebildet. Eine ergänzende Anpassung oder Korrektur der Form der Knochenplatte 1 kann vom Nutzer unmittelbar vor der Implantation vorgenommen werden, indem insbesondere die Biegezonen 7, 8 im Bereich der Materialtaillierungen 5, 6 genutzt werden.

Gemäß den Darstellungen in den Fig. 1 und 2 ist die Knochenplatte 1 mit einer umlaufenden Phase oder Verrundung 16 versehen.

Die Knochenplatte 1 weist im mittleren Plattenabschnitt 4 sowie im proximalen Plattenabschnitt 3 jeweils einen Durchbruch 17, 18 auf. Die Durchbrüche 17, 18 dienen der Aufnahmen eines jeweiligen Kirschner-Drahtes, was unten in den Fig. 3 und 5 gezeigt ist. Die Kirschner-Drähte können vom Operateur als Hilfsmittel während der Implantation der Knochenplatte 1 genutzt werden.

Die Fig. 3 bis 5 zeigen perspektivische Darstellungen eines Bereiches eines Fußskeletts mit einem Tarsometatarsal-I-Gelenk 30, welches zur Versteifung mittels der Knochenplatte 1 aus den Fig. 1 und 2 gesichert ist. Zu diesem Zweck sind nach dem Fixieren der Knochenplatte 1 in die distale Bohrung 9 und die weitere distale Bohrung 10 eine jeweilige Knochenschraube (nicht dargestellt) winkelstabil eingeschraubt. Die Winkelstabilität der eingeschraubten Knochenschrauben wird hierbei dadurch erreicht, dass ein konisches Gewinde am jeweiligen Schraubenkopf in zugeordnetes Gewinde in der distalen und der weiteren distalen Bohrung 9, 10 eingreift. Die Knochenschrauben im distalen Plattenabschnitt 2 sind divergierend eingeschraubt.

Auch in die plantare und die nach medial abgewickelte Bohrung 11, 12 ist eine jeweilige Knochenschraube 31, 32 eingeschraubt, wobei die beiden Knochenschrauben 31, 32 sich gemäß den Darstellungen in den Fig. 3 und 4 im Knochen kreuzen.

Aus den Fig. 3 und 4 ergibt sich weiterhin, dass Kirschner-Drähte 33, 34, die in die Durchbrüche eingeführt sind, jeweils im Wesentlichen achsenparallel zu einer der Knochenschrauben verlaufen.

## Patentansprüche

1. Anatomisch angepasste, plantare Knochenplatte (1) zur winkelstabilen Fixation für die Arthrodese des Tarsometatarsal-I-Gelenks, mit:
- einem distalen Plattenabschnitt (2), der sich in Längsrichtung erstreckt und in welchem eine distale Bohrung (9) zum winkelstabilen Aufnehmen einer Knochenschraube (31) gebildet ist,
- einem proximalen Plattenabschnitt (3), der sich quer zur Längsrichtung erstreckt, und
- einem mittleren Plattenabschnitt (4), welcher sich zwischen dem distalen und dem proximalen Plattenabschnitt (2, 3) erstreckt,
wobei im proximalen Plattenabschnitt (3) quer zur Längsrichtung nebeneinander eine plantare Bohrung (11) zum winkelstabilen Aufnehmen einer plantar zu fixierenden Knochenschraube (34) und eine nach medial abgewinkelte Bohrung (12) zum winkelstabilen Aufnehmen einer nach medial abgewinkelt zu fixierenden Knochenschraube (35) angeordnet sind, **dadurch gekennzeichnet, dass** die Knochenplatte (1) an die anatomischen Verhältnisse im Bereich des Tarsometatarsal-I-Gelenks angepasst ist, indem
- die nach medial abgewinkelte Bohrung (12) an einem seitlich abstehenden Abschnitt (13) des proximalen Plattenabschnitts (3) gebildet ist,
- der seitlich abstehende Abschnitt (13) gegenüber einem mittleren Abschnitt (14) des proximalen Plattenabschnittes (3) zur Plattenunterseite hin geneigt und mit einer Schränkung versehen ist,
- der mittlere Plattenabschnitt (4) frei von Schraubenbohrungen ist,
- im Bereich des proximalen Plattenabschnitts (3) ein im Wesentlichen ebener Plattenabschnitt gebildet ist, der sich zu dem mittleren Plattenabschnitt (4) hin fortsetzt,
- der mittlere Plattenabschnitt (4) mit einem gebogenen Plattenabschnitt mit einer proximalen Biegezone (8) gebildet ist, die dem Gelenkspalt des zu fixierenden Gelenks zugeordnet ist und sich an den im Wesentlichen ebenen Plattenabschnitt im Bereich des proximalen Plattenabschnitts (3) anschließt, und
- der mittlere Plattenabschnitt über eine weitere Biegezone oder Biegung (7) an den distalen Plattenabschnitt (2)) koppelt,
wobei die Biegezonen (7;8) jeweils mit einer Materialverdünnung (5; 6) gebildet sind, so dass sie zur ergänzenden Formanpassung der Knochenplatte (1) an anatomische Verhältnisse nutzbar sind, indem vorgegebene Biegungen veränderbar sind.

2. Knochenplatte nach Anspruch 1, dadurch **gekennzeichet,** dass mittels des distalen und des proximalen Plattenabschnitts (2, 3) und des sich hierzwischen erstreckenden mittleren Plattenabschnitts (4) in Blickrichtung von der Seite eine S-Plattenkontur gebildet ist.

3. Knochenplatte nach mindestens einem der vorangehenden Ansprüche, **dadurch gekennzeichnet, dass** die plantare Bohrung (11) und die nach medial abgewinkelte Bohrung (12) mit sich auf der Plattenunterseite kreuzenden Einschraubachsen für die plantar und die nach medial abgewinkelt zu fixierenden Knochenschrauben (34, 35) gebildet sind.

4. Knochenplatte nach mindestens einem der vorangehenden Ansprüche, **dadurch gekennzeichnet, dass** im distalen Plattenabschnitt (2) in Längsrichtung benachbart zu der distalen Bohrung (9) eine weitere distale Bohrung (10) zum winkelstabilen Aufnehmen einer weiteren Knochenschraube (32) gebildet ist.

5. Knochenplatte nach mindestens einem der vorangehenden Ansprüche, **gekennzeichnet durch** ein oder mehrere Durchbrüche (17; 18) zum winkelstabilen Aufnehmen eines Kirschner-Drahtes (36; 37).

6. Knochenplatte nach Anspruch 5, **dadurch gekennzeichnet, dass** eine Einsteckachse eines Durchbruchs (18), der im proximalen Plattenabschnitt (3) oder benachbart hierzu gebildet ist, im Wesentlichen achsenparallel zur Einschraubachse für die plantare Knochenschraube (34) in der plantaren Bohrung (11) verläuft.

7. Knochenplattensystem für die Arthrodese des Tarsometatarsal-I-Gelenks, mit:
- einer anatomisch angepassten, plantaren Knochenplatte (1), bei der
- ein distaler Plattenabschnitt (2), der sich in Längsrichtung erstreckt und in welchem eine distale Bohrung (9) zum winkelstabilen Aufnehmen einer Knochenschraube (31) gebildet ist,
- eine proximaler Plattenabschnitt (3), der sich quer zur Längsrichtung erstreckt, und
- eine mittlerer Plattenabschnitt (4) gebildet sind, welcher sich zwischen dem distalen und dem proximalen Plattenabschnitt (2, 3) erstreckt,
wobei im proximalen Plattenabschnitt (3) quer zur Längsrichtung nebeneinander eine plantare Bohrung (11) zum winkelstabilen Aufnehmen einer plantar zu fixierenden Knochenschraube (34) und eine nach medial abgewinkelte Bohrung (12) zum winkelstabilen Aufnehmen einer nach medial abgewinkelt zu fixierenden Knochenschraube (35) angeordnet sind, und
- mehreren Knochenschrauben (31, 34, 35) zum jeweils winkelstabilen Einschrauben in die distale Bohrung (9), die plantare Bohrung (11) sowie die mediale Bohrung (12),
**dadurch gekennzeichnet, dass** die Knochenplatte (1) an die anatomischen Verhältnisse im Bereich des Tarsometatarsal-I-Gelenks angepasst ist, indem
- die nach medial abgwinkelte Bohrung (12) an einem seitlich abstehenden Abschnitt (13) des proximalen Plattenabschnitts (3) gebildet ist,
- der seitlich abstehende Abschnitt (13) gegenüber einem mittleren Abschnitt (14) des proximalen Plattenabschnittes (3) zur Plattenunterseite hin geneigt und mit einer Schränkung versehen ist,
- der mittlere Plattenabschnitt (4) frei von Schraubenbohrungen ist,
- im Bereich des proximalen Plattenabschnitts (3) ein im Wesentlichen ebener Plattenabschnitt gebildet ist, der sich zu dem mittleren Plattenabschnitt (4) hin fortsetzt,
- der mittlere Plattenabschnitt (4) mit einem gebogenen Plattenabschnitt mit einer proximalen Biegezone (8) gebildet ist, die dem Gelenkspalt des zu fixierenden Gelenks zugeordnet ist und sich an den im Wesentlichen ebenen Plattenabschnitt im Bereich des proximalen Plattenabschnitts (3) anschließt, und
- der mittlere Plattenabschnitt über eine weitere Biegezone oder Biegung (7) an den distalen Plattenabschnitt (2)) koppelt,
wobei die Biegezonen (7;8) jeweils mit einer Materialverdünnung (5; 6) gebildet sind, so dass sie zur ergänzenden Formanpassung der Knochenplatte (1) an anatomische Verhältnisse nutzbar sind, indem vorgegebene Biegungen veränderbar sind.

## Claims

1. An anatomically adapted plantar bone plate (1) for fixation of the arthrodesis of the tarsometatarsal I joint in a stable angle, comprising:
- a distal plate section (2), which extends in the longitudinal direction, and in which a distal bore (9) is formed for receiving a bone screw (31) at a stable angle,
- a proximal plate section (3), which extends transversely to the longitudinal direction, and
- a central plate section (4), which extends between the distal plate section and the proximal plate section (2, 3),
wherein a plantar bore (11) for receiving a bone screw (34) to be affixed in a plantar position at a stable angle and a bore (12) at a medial angle for receiving a bone screw (35) to be affixed at a stable angle in the medial direction are arranged side-by-side transversely to the longitudinal direction in the proximal plate section (3),
**characterized in that** the bone plate (1) is adapted to the anatomical conditions in the area of the tarsometatarsal I joint, **in that**
- the bore (12) that is angled medially is formed on a laterally protruding section (13) of the proximal plate section (3),
- the laterally protruding section (13) is inclined towards the bottom side of the plate and is provided with an offset relative to a central section (14) of the proximal plate section (3),
- the central plate section (4) is free of bore holes for screws,
- an essentially planar plate section that continues towards the central plate section (4) is formed in the area of the proximal plate section (3),
- the central plate section (4) is formed with a curved plate section with a proximal bending zone (8) that is assigned to the joint gap of the joint to be affixed and that is connected to the essentially planar plate section in the area of the proximal plate section (3), and
- the central plate section is connected to the distal plate section (2) by way of another bending zone or bend (7),
wherein the bending zones (7; 8) are each formed with a thinning of material (5; 6), so that they can be used for additional adaptation of the shape of the bone plate (1) to anatomical conditions, **in that** predetermined bends are adjustable.

2. The bone plate according to claim 1, **characterized in that** by means of the distal and proximal plate sections (2, 3) and the central plate section (4) extending there between, a plate contour having an S shape when seen from the side is formed.

3. The bone plate according to at least one of the preceding claims, **characterized in that** the plantar bore (11) and the bore (12), which is angled medially, are formed with screw-in axes, intersecting on the bottom side of the plate, for the bone screws (34, 35) that are plantar and to be affixed at a medial angle.

4. The bone plate according to at least one of the preceding claims, **characterized in that** another distal bore (10) for receiving an additional bone screw (32) at a stable angle is formed in the distal plate section (2) in the longitudinal direction adjacent to the distal bore (9).

5. The bone plate according to at least one of the preceding claims, **characterized by** one or more breakthroughs (17; 18) for receiving a Kirschner wire (36; 37) at a stable angle.

6. The bone plate according to claim 5, **characterized in that** an insertion axis of a breakthrough (18) that is formed in or adjacent to the proximal plate section (3) proceeds essentially axially parallel to the screw-in axis for the plantar bone screw (34) in the plantar bore (11).

7. A bone plate system for arthrodesis of the tarsometatarsal I joint, comprising
- an anatomically adapted plantar bone plate (I), in which
- a distal plate section (2), which extends in the longitudinal direction, and in which a distal bore (9) is formed to receive a bone screw (31) at a stable angle,
- a proximal plate section (3), which extends transversely to the longitudinal direction, and
- a central plate section (4) are formed, extending between the distal and proximal plate sections (2, 3),
wherein a plantar bore (11) for receiving a bone screw (34) to be affixed at a stable angle on the plantar side and a bore (12) at a medial angle for receiving a bone screw (35) to be affixed at a stable angle in the medial direction are arranged side-by-side transversely to the longitudinal direction in the proximal plate section (3), and
- a plurality of bone screws (31, 34, 35), each for screwing into the distal bore (9), the plantar bore (11) and the medial bore (12) at a stable angle,
**characterized in that** the bone plate (1) is adapted to the anatomical conditions in the area of the tarsometatarsal I joint, **in that**
- the bore (12) that is angled medially is formed on a laterally protruding section (13) of the proximal plate section (3),
- the laterally protruding section (13) is inclined towards the bottom side of the plate and is provided with an offset relative to a central section (14) of the proximal plate section (3),
- the central plate section (4) is free of boreholes for screws,
- an essentially planar plate section that continues towards the central plate section (4) is formed in the area of the proximal plate section (3),
- the central plate section (4) is formed with a curved plate section with a proximal bending zone (8) that is assigned to the joint gap in the joint to be affixed and that is connected to the essentially planar plate section in the area of the proximal plate section (3), and
- the central plate section is connected to the distal plate section (2) by way of another bending zone or bend (7),
wherein the bending zones (7; 8) are each formed with a thinning of material (5; 6), so that they can be used for additional adaptation of the shape of the bone plate (1) to anatomical conditions, **in that** predetermined bends are adjustable.

## Revendications

1. Plaque d'os plantaire, anatomiquement adaptée (1) pour la fixation à angle fixe pour l'arthrodèse de la 1^{ère} articulation tarso-métatarsienne avec :
- une section de plaque distale (2), qui s'étend dans la direction longitudinale et dans laquelle est formé un perçage distal (9) pour recevoir à angle fixe une vis à os (31),
- une section de plaque proximale (3), qui s'étend transversalement à la direction longitudinale, et
- une section de plaque centrale (4), laquelle s'étend entre la section de plaque distale et la section de plaque proximale (2, 3),
un perçage plantaire (11) pour loger à angle fixe une vis à os (34) à fixer au niveau plantaire et un perçage (12) incliné en direction médiale pour recevoir à angle fixe une vis à os à fixer inclinée en direction médiale (35) étant disposés dans la section de plaque proximale (3) l'un à côté de l'autre, transversalement à la direction longitudinale **caractérisée en ce que** la plaque d'os (1) est adaptée aux conditions anatomiques dans la zone de la 1^{ere} articulation tarso-métatarsienne,
- le perçage incliné en direction médiale (12) étant formé sur une section s'écartant latéralement (13) de la section de plaque proximale (3),
- la section s'écartant latéralement (13) étant inclinée par rapport à une section centrale (14) de la section de plaque proximale (3) vers la face inférieure de la plaque et étant dotée d'un décalage,
- la section de plaque centrale (4) étant dépourvue de perçages de vis,
- une section de plaque pour l'essentiel plane étant formée dans la zone de la section de plaque proximale (3) qui continue vers la section de plaque centrale (4),
- la section de plaque centrale (4) avec une section de plaque recourbée étant formée avec une zone de cambrure proximale (8), qui est affectée à l'interligne articulaire de l'articulation à fixer et se raccorde à la section de plaque pour l'essentiel plane dans la zone de la section de plaque proximale (3), et
- la section de plaque centrale s'accouplant par le biais d'une autre zone de cambrure ou cambrure (7) à la section de plaque distale (2),
les zones de cambrure (7 ; 8) étant respectivement formées avec un amincissement de matériau (5 ; 6) de telle sorte qu'elles peuvent être utilisées pour l'adaptation de forme à compléter de la plaque d'os (1) aux conditions anatomiques, les cambrures prédéfinies pouvant être modifiées.

2. Plaque d'os selon la revendication 1, **caractérisée en ce qu'**un profil de plaque en S est formé, dans le sens d'observation depuis le côté, au moyen de la section de plaque distale et de la section de plaque proximale (2, 3) et de la section de plaque centrale s'étendant entre elles (4).

3. Plaque d'os selon au moins l'une quelconque des revendications précédentes, **caractérisée en ce que** le perçage plantaire (11) et le perçage incliné en direction médiale (12) sont formés avec des axes de vissage se croisant sur la face inférieure de la plaque pour les vis à os (34, 35) à fixer au niveau plantaire et inclinées en direction médiale.

4. Plaque d'os selon au moins l'une quelconque des revendications précédentes, **caractérisée en ce qu'**un autre perçage distal (10) est formé dans la section de plaque distale (2) dans la direction longitudinale de façon contigüe au perçage distal (9) pour recevoir à angle fixe une autre vis à os (32).

5. Plaque d'os selon au moins l'une quelconque des revendications précédentes, **caractérisée par** une ou plusieurs percées (17 ; 18) pour recevoir à angle fixe une broche de Kirschner (36 ; 37).

6. Plaque d'os selon la revendication 5, **caractérisée en ce qu'**un axe d'enfichage d'une percée (18), qui est formé dans la section de plaque proximale (3) ou contigu à celle-ci, passe pour l'essentiel axialement parallèle à l'axe de vissage pour la vis à os plantaire (34) dans le perçage plantaire (11).

7. Système de plaque d'os pour l'arthrodèse de la 1^{ère} articulation tarso-métatarsienne, avec :
- une plaque d'os plantaire, anatomiquement adaptée (1), pour laquelle sont formées
- une section de plaque distale (2), qui s'étend dans la direction longitudinale et dans laquelle est formé un perçage distal (9) pour recevoir à angle fixe une vis à os (31),
- une section de plaque proximale (3), qui s'étend transversalement à la direction longitudinale, et
- une section de plaque centrale (4), laquelle s'étend entre la section de plaque distale et la section de plaque proximale (2, 3),
un perçage plantaire (11) pour loger à angle fixe une vis à os (34) à fixer au niveau plantaire et un perçage (12) incliné en direction médiale pour recevoir à angle fixe une vis à os à fixer inclinée en direction médiale (35) étant disposés dans la section de plaque proximale (3) l'un à côté de l'autre, transversalement à la direction longitudinale, et
- plusieurs vis à os (31, 34, 35) pour visser respectivement à angle fixe dans le perçage distal (9), le perçage plantaire (11) ainsi que dans le perçage médial (12),
**caractérisée en ce que** la plaque d'os (1) est adaptée aux conditions anatomiques dans la zone de la 1^{ère} articulation tarso-métatarsienne,
- le perçage incliné en direction médiale (12) étant formé sur une section s'écartant latéralement (13) de la section de plaque proximale (3),
- la section s'écartant latéralement (13) étant inclinée par rapport à une section centrale (14) de la section de plaque proximale (3) vers la face inférieure de la plaque et étant dotée d'un décalage,
- la section de plaque centrale (4) étant dépourvue de perçages pour vis,
- une section de plaque pour l'essentiel plane étant formée dans la zone de la section de plaque proximale (3) qui continue vers la section de plaque centrale (4),
- la section de plaque centrale (4) avec une section de plaque recourbée étant formée avec une zone de cambrure proximale (8), qui est affectée à l'interligne articulaire de l'articulation à fixer et se raccorde à la section de plaque pour l'essentiel plane dans la zone de la section de plaque proximale (3), et
- la section de plaque centrale s'accouplant par le biais d'une autre zone de cambrure ou cambrure (7) à la section de plaque distale (2),
les zones de cambrure (7 ; 8) étant respectivement formées avec un amincissement de matériau (5 ; 6) de telle sorte qu'elles peuvent être utilisées pour l'adaptation de forme à compléter de la plaque d'os (1) aux conditions anatomiques, les cambrures prédéfinies pouvant être modifiées.
